# EUROPEAN PATENT SPECIFICATION

(11) **EP 3 666 475 B1**
(45) Date of publication and mention of the grant of the patent: **09.08.2023**
(21) Application number: 19212242.2
(22) Date of filing: 28.11.2019
(51) Int. Cl.: B25J 9/06

(54) **CONTINUUM ROBOT**
KONTINUUMROBOTER
ROBOT CONTINUUM

(30) Priority: 14.12.2018 GB 201820398
(43) Date of publication of application: 17.06.2020
(73) Proprietor: Rolls-Royce plc, London N1 9FX (GB)
(72) Inventor: Dong, Xin, Derby, Derbyshire DE24 8BJ (GB); Norton, Andrew, Derby, Derbyshire DE24 8BJ (GB); Lowth, Stewart, Derby, Derbyshire DE24 8BJ (GB)
(74) Representative: Rolls-Royce plc

(56) References cited:
- WO-A1-95/18311
- US-A- 4 753 222
- US-A- 5 005 558
- US-A1- 2005 131 279
- US-A1- 2006 156 851
- US-A1- 2008 214 897
- US-A1- 2014 058 364
- US-A1- 2016 279 789

## Description

### Field of the Disclosure

The invention relates to a continuum robot in particular for the design of an arm section of a continuum robot. This section can be part or a number of sections that form a full length arm that is capable of the inspection or repair of a component in a confined or dangerous environment.

### Background of the Disclosure

The use of robotics in manufacture, inspection and repair of complex structures is growing as these devices are able to access points that humans are unable to access. In order to perform such tasks there are a large number of robot designs and configurations, which are desirable for different purposes. In particular, snake arm or continuum robots are of particular interest. These continuum robots have numerous degrees of freedom that allow them to be particularly suited to accessing confined spaces unlike conventional rigid link robots. This allows them to be used for a wide range of purposes from surgery to in-situ repair of aero engines.

Continuum robots generally consist of several independently controlled sections, with each section having one or two degrees-of-freedom which allows them to be easily manipulated. Each element of the sections is connected by rigid or compliant joints, which can be manipulated by pulling cables or adjusting pneumatic or hydraulic actuators; thus, allowing them to be articulated to different targeted orientations. One of the most common designs of continuum robots consists of using rigidly linked sections coupled together using revolute/universal/spherical (R/U/S) joints. There are, however, issues with the use of such systems; this is because of the difficulty in running a delivery tube with a diameter close to the inner diameter of the arm through the R/U/S joint. The issue is the result of the bending radius of the joint being small. This small radius can result in a kink being formed in the delivery tube, which can block the functioning of the tool or the delivery of material along the tube. In order to overcome this, a continuum section can feature an elastomer section between the rigid links; this is used to distribute the bend evenly over the whole length of the section. Consequently, use of an elastomer section allows for a more continuous curvature, which allows for the delivery tube to run through the middle of the robot arm. Thus, by employing elastomer materials within the sections of a continuum robot allows the robot's arm to have better bending capability, when compared to a rigid-joint continuum arm.

One of the issues, however, of this type of robot is that when controlling the robot's arm using a kinematic model is that sections are considered as circular curves. In this case the kinematic model provides a means of controlling/determining the motion of multiple linked bodies. However, physical tests demonstrate that the continuum sections do not bend with a continuous and circular radius of curvature. Therefore, the discrepancy between the model and the actual shape of the arm can cause considerable amounts of positional errors for the robot. Consequently, there is a need to make the continuum section to better match circular curvature, such that the control of the continuum robot is more reliable.

United States Patent Application US 2014/058364 A1 discloses a section of a robotic arm having a decreasing tension along the length of the arm. The document discloses the use of different spring tensions, or of shaped joints, to provide this change in tension. United States Patent Application US 2005/131279 A1 discloses an arm for an endoscope having a number of joints with the same stiffness. United States Patent Application US 2016/214897 A1 describes an arm for a continuum robot having alternating rigid and flexible members to control the bending of the arm. United States Patent Application US 2008/214897 A1 discloses an endoscope device having an outer cover that has a thickness that varies such that the rigidity increases along the length of the endoscope. United States Patent Application US 4753222 A discloses a flexible tube for covering an endoscope. International Patent Application WO 95/18311 A1 (D6) discloses a continuum arm robot which has varying stiffness along the length of the arm; this change in stiffness is done through the use of rigid segments.

### Summary of the Disclosure

The scope of the protection is set out in the appended claims.

According to a first aspect there is provided a continuum arm robot section, in which the section comprises at least three segments, featuring a base, at least one middle segment and a tip segment, with each segment corresponding to at least one interconnecting portion between pairs of neighbouring discs that form a backbone of the section of the continuum arm robot, wherein the base segment having a greater stiffness than the at least one middle segment and tip segment and the at least one middle segment having a greater stiffness than the tip segment.

By employing the teachings of the disclosure, the continuum robot can have sections that curve with a circular shape when bent, and also be adapted to form other bending shapes, such as quadratic curves. As such, this would provide the robot with better accessibility when navigating within confined spaces, or in situations where extreme control of the arm is required. Furthermore, as these sections are of set design, they can be adjusted on site to improve the performance of the robot arm.

Each segment is made from a compliant and revolute joint pair.

The stiffness of the joints is determined by the surface roughness of the revolute joint.

The stiffness of the joints may be determined by the material of the discs within the revolute joints.

At least one sleeve may be provided around a segment in order to adjust the stiffness of said section.

The sleeve sections may be made from one or more of thermoplastic materials, shape memory alloys and shape memory polymers.

At least one stiffening member may be provided on a segment in order to adjust the stiffness of said section.

The stiffening members may be made from one or more of thermoplastic materials, shape memory alloys and shape memory polymers.

According to a second aspect there is provided a continuum robot having one or more arms, each arm comprising at least one continuum robot arm section as defined above.

The continuum robot may be used in the inspection and/or repair of a gas turbine engine.

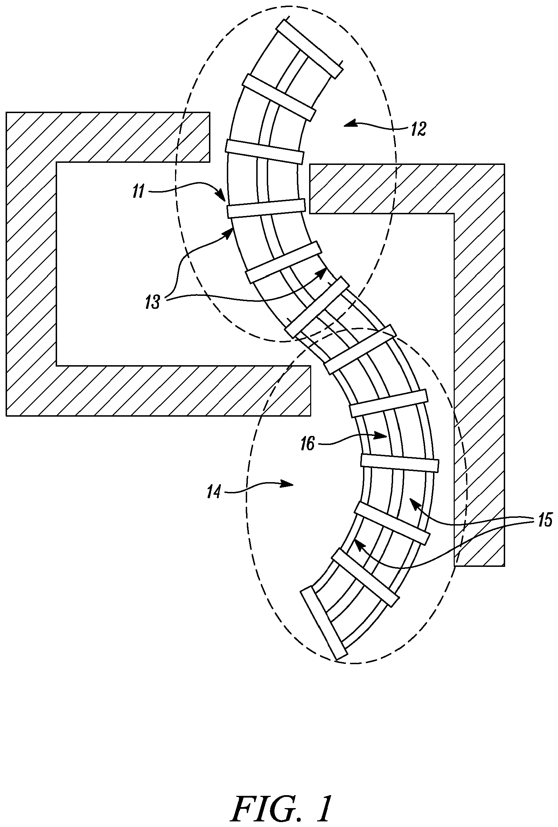

In this text a continuum robot is a hyper-redundant robot having a large or infinite number of degrees of freedom. These robots act as a continuously curving manipulator that is self-supporting. In order to fulfil these requirements, the robot is made from individual sections, each of which are controlled by different sets of tendons. An example of such a continuum robot is shown in **Figure 1**. The continuum robot has two sections; a first section 12 and a second section 14. The first section 12 is controlled by cables 13, whilst the second section 14 is controlled by cables 15. The cables in continuum arm robots are constrained within the holes of the disk components 11. By pulling tendons 16, each of the sections are able to move relative to each other such that the robot can be manipulated. The movement of the cables is actuated by motor/artificial muscle.

### Brief description of the drawings

Embodiments will now be described by way of example only, with reference to the Figures, in which:
**Figure 1** is a prior art example of a part of a continuum arm robot featuring two individual arm sections;
**Figure 2** is a prior art example of a simple compliant joint structure;
**Figures 3A-3D** are a prior art examples of a continuum section for use in a continuum arm robot;
**Figures 4A**-**B** present two embodiments of a continuum section having a two-degrees-of-freedom which don't represent the invention;

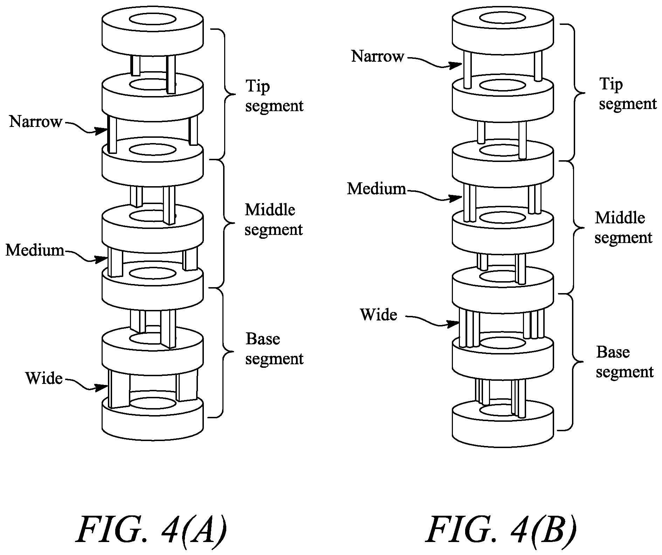

**Figure 4A** presents an example using a single piece interconnect, **Figure 4B** presents an example wherein the interconnect is formed from a number of interconnect parts;

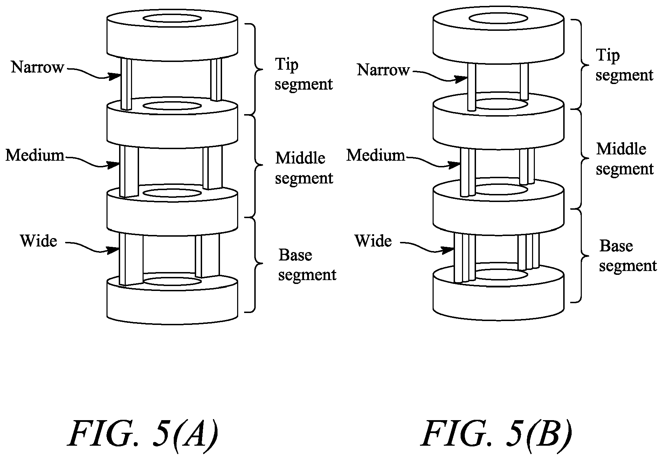

**Figures 5A-B** presents two embodiments of a continuum section having a single-degree-of-freedom which don't represent the invention; **Figure 5A** presents an example using a single piece interconnect, **Figure 5B** presents an example wherein the interconnect is formed from a number of interconnect parts;

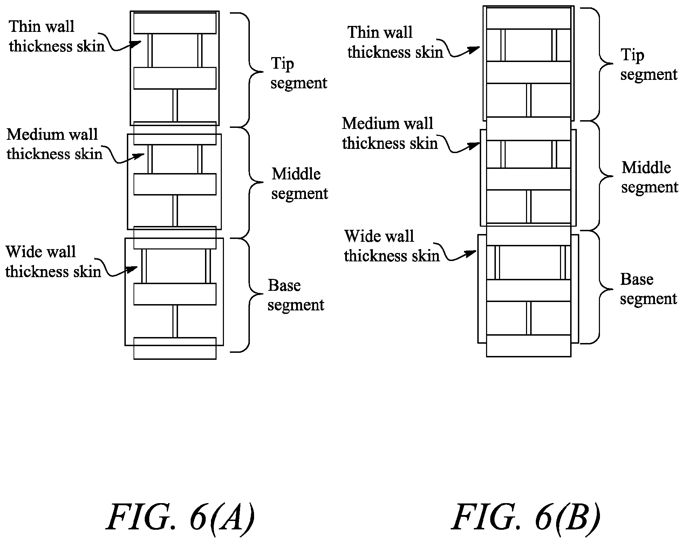

**Figures 6A-B** presents an embodiment of a continuum section having a two-degrees-of-freedom which doesn't represent the invention, wherein a wall section is provided for each of the separate segments; **Figure 6A** presents an outer view of the embodiment, whilst **Figure 6B** presents a cut-away view;

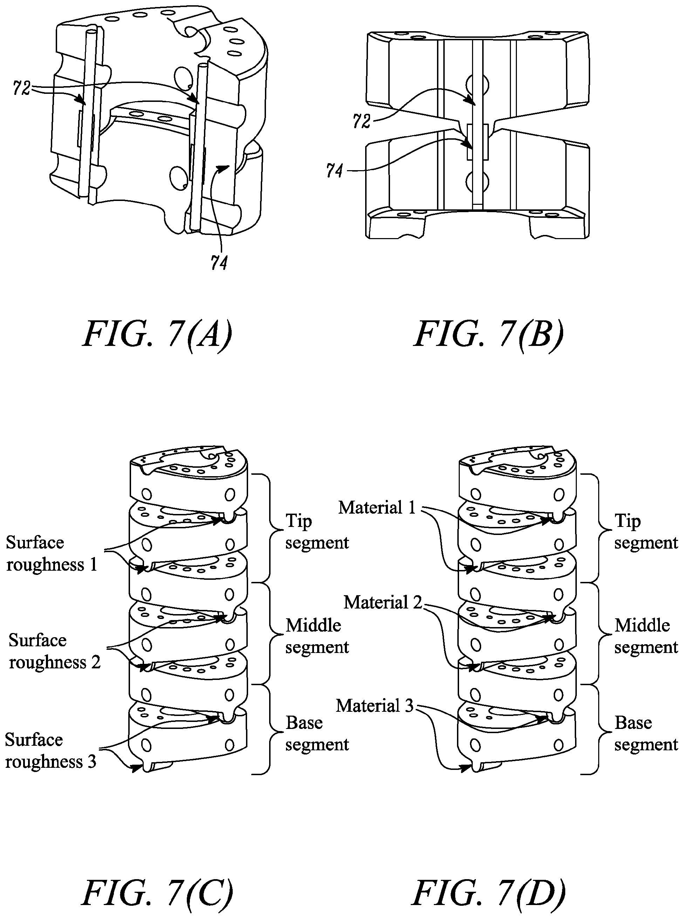

**Figures 7A-D** present two embodiment of a continuum section comprising rigid revolute joints as well as compliant joints; **Figure 7A** presents a cut away of a single segment of such a joint, whilst **Figure 7B** presents an outside view of the joint structure; **Figures 7C** presents an embodiment of the present disclosure wherein the revolute joints have different surface roughness; and **Figure 7D** presents an embodiment of the present disclosure wherein the revolute joints have different materials having differing materials of the discs.

### Detailed Description

Aspects and embodiments of the present disclosure will now be discussed with reference to the corresponding drawings. Other aspects and embodiments will be apparent to those skilled in the art.

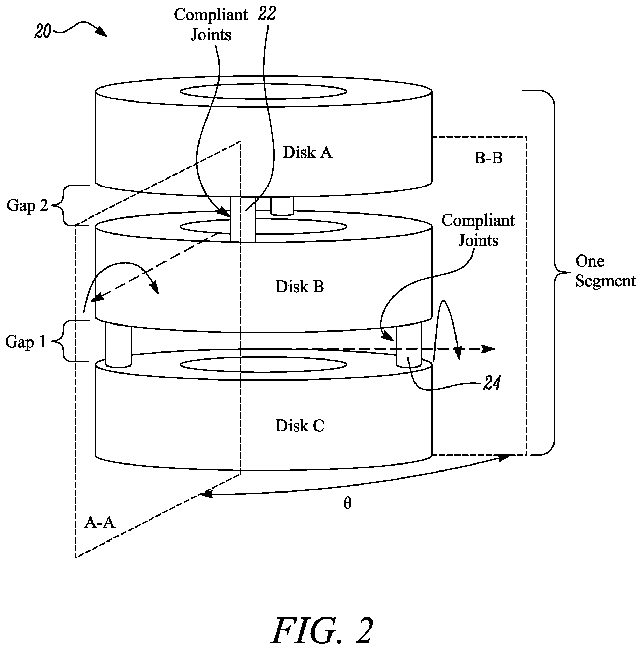

**Figure 2** displays a prior art schematic of a twin compliant joint structure 20. In this there are three discs (A, B, C) which are coupled together using compliant joints 22 and 24, such that a first compliant joint 22 links disc A to disc B, and a second compliant joint 24 connects disc B to disc C. The two compliant joints are angled relative to each other, such that they are rotated by 90° relative to each other. This configuration of a section allows for a contraction/extension along the lines A-A by manipulation of the compliant joints between discs B and C, whilst movement of the joint can be made along the lines B-B by manipulation of the compliant joint between discs A and B. Manipulation of the joint can be done by the movement of cables or actuators which are connected to the discs. By controlled motion, for example by the pulling or pushing of the cables or actuators that are attached to the disc, results in the manipulation of the discs. This allows a continuum arm robot with a number of these sections to be able to move in multiple degrees of freedom and thus allows for movement along complex passages and to work on difficult to reach objects. The movement of the cables are pulled using a series of proximal motors positioned outside of the restricted area. Controlled linear or rotational movement of the motors, in open or closed loop control using in-built sensors - for example: positional or force - corresponds with accurate movements of the cables and thus the continuum robot arm discs. The movement of the arm can thus be controlled by a computer program operating a kinematic model. These computer programs for manipulating a continuum arm include both forward and inverse kinematic models. In most of these models the shape of section of continuum robot is considered as having circular curvature. This modelling is chosen as it makes the mathematics of the shape are easier, and thus requires less computing time to derive the required movement to position the arm. As discussed above, without greater improvement of the arm sections such modelling is unsuitable and either requires greater complexity of the modelling and thus more computing time, or any movement in such an arm results in an inherent lack of accuracy of the robot arm, which could damage any device that is being inspected or being worked on and/or the robot arm.

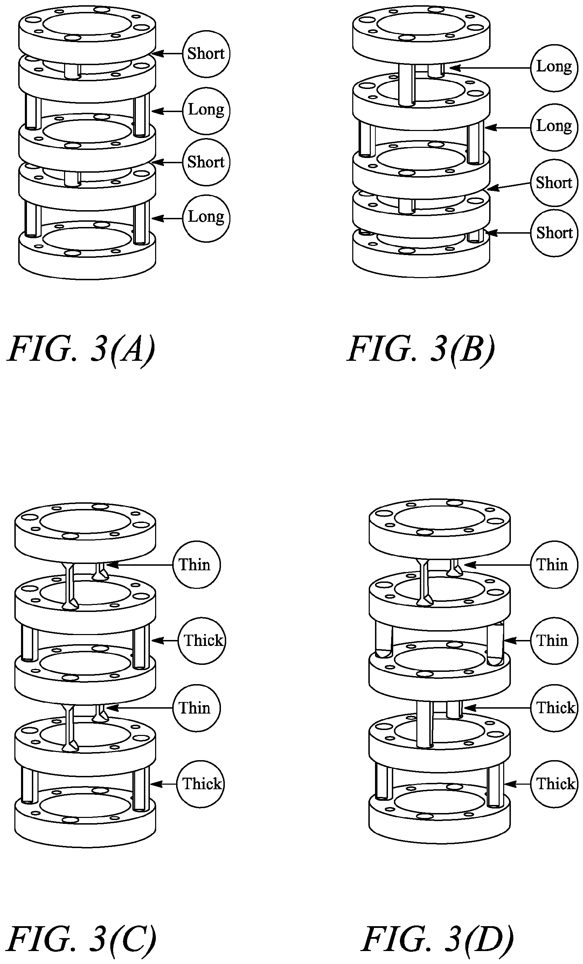

Examples of a section of a prior art continuum section for use in a robot is shown in **Figure 3A and 3B**. Here the multiple discs that form the section are linked by either alternating short and long elastomeric joints. The different length of the joints provides different stiffness for the sections, so the short sections bend less than the long sections. Alternatively, thin and thick section or thin-thin-thick-thick joints, which have the same length, have also been used as shown in **Figures 3C and 3D****.** This again results in each segment of the section having certain stiffness with the thicker sections being stiffer than the thinner sections. These do not overcome the issues presented above that the radius of curvature is nonideal, and as such can lead to issues with the delivery tube that runs through the centre of the joint sections.

An improvement to using these alternating thick or thin interconnecting sections between the discs can be achieved by allowing the stiffness to be adjusted along the length of the section. This is done by separating the length of the continuum section into three or more segments, such that they form at least a tip segment, a middle segment and a base segment. Each of these separate segments is provided with disc interconnects having different stiffness levels. To achieve this, the base segment has a wide interconnect between the discs in the base segment providing it with greater degree of rigidity. The middle section may be provided with disc interconnects of medium thicknesses. Whilst the tip segment is then provided with the narrowest interconnect between the discs. By having a wide interconnect at the base, with progressively thinner sections for the middle and the tip segments provides these three segments with different levels of stiffness. With the base having the highest level of stiffness the base will prevent the section from kinking at the start allowing for a tube to be easily contained within it. These interconnects can either be of a single piece construction as is presented in **Figure 4A****.** Alternatively, the interconnects may be formed of a number of parts having a standard thickness. Therefore, in order to achieve the varying thickness of the segments the interconnects can be added together such that the base segment has three interconnect parts coupled together to form a full interconnect structure, as presented in **Figure 4B****.** The middle segment may have two interconnect parts coupled together to form the complete interconnect. The tip section having the lowest stiffness may only feature a single interconnect part. However, as the person skilled in the art will appreciate, this could be any suitable number of interconnect parts for each segment. Each of the segments can be bent in 2 degree-of-freedom.

In the above example the segment has two interconnecting stages per segment, however, as shown in **Figure 5** it is possible to achieve the same effect, for a single degree of freedom joint, with only a single interconnect forming each segment. In this case the interconnect parts can either be a single unit (**Figure 5A**) or can be formed from a number of interconnect parts coupled together (**Figure 5B**), as described above. In this case the base segment has the highest stiffness and may feature a wide interconnect section, or a greater number of interconnect parts. The middle segment being narrower or having a smaller number of interconnect parts. Finally, the tip section is narrower than the other sections or features the fewest number of interconnect parts.

Although the embodiment has been presented in terms of thickness of the interconnects, similar effects could also be produced by using materials having different stiffness properties. This could be achieved using the same thickness interconnect, but with the interconnect being made using a material that has a greater level of stiffness, than the middle segment's interconnects, which in turn is greater than that of the interconnect in the tip segment. Examples of such materials that could be employed are titanium 64 alloy (105-120 GPa), aluminium (69 GPA), steel (200 GPa), tungsten (400 GPa), silicon carbide (450 GPz). Alternatively, materials such as nylon (4 GPa), PET (2 GPa) or carbon reinforced plastics (50 GPa) could be considered for devices requiring less rigidity/stiffness.

An alternative embodiment of this disclosure is that rather than changing thickness or stiffness of the interconnects is to provide walls for the discs and interconnects, which form the three separate segments that form the complete section that forms a part of a continuum arm. The wall portions for each of these separate segments are provided with a different stiffness, such that the base segment is stiffer than the middle, which is in turn stiffer than the tip. An example of this is shown in **Figures 6A and 6B**. In this the materials for each of these walls are the same, and the wall sections are of different thickness such that the base section features a wide wall section, the middle segment having a medium wall thickness, and a tip section, which has a thin wall section. Suitable materials for the wall section may be elastic nitinol, spring steel. Alternatively, plastics or rubber materials could be used. The wall sections can be made from materials having different stiffness levels, such that the stiffness of the wall sections decreases in these segments of the section from base to tip.

An alternative embodiment utilises revolute and compliant joints integrated into a single section and to adjust the stiffness along the longitudinal axis. An example of such a single joint is shown in **Figure 7A****.** The single component is shown having a compliant joint part 72 and the rigid revolute joint 74. In this embodiment, along the longitudinal axis the stiffness of the compliant joint is constant, whilst the revolute joint stiffness can be varied. By combining these revolute and compliant joint segments a section be formed. An example of this as in the present disclosure is shown in **Figure 7B****.** This figure presents an example in which three of these rigid revolute and compliant joints form a single joint section. Each of these joint sections are formed having a base segment, middle segment and tip segment. In this the revolute joints between the sections are provided with joints having different surface roughness or being made of different materials. By varying the surface roughness of the joints means that the pliability of the joints can be varied, which varies the stiffness of the joints. In this disclosure the base segment has the greatest surface roughness, so that this joint is the stiffest. The middle segment has a medium surface roughness and thus forming a middle stiffness joint. The tip segment has the lowest surface roughness and as such forms the least stiff joint. The reference to the surface roughness and the stiffness of the joints are when compared to each of the other joints. For example, the surface roughness of the middle segment may have 10-99% of the surface roughness of base segment. The tip segment may have 10-99% of the surface roughness of the middle segment. This roughness difference may be in the range of about 10-75% for both the base to middle section and then again for the middle to the tip segments respectively. An alternative to using the joints having different surface roughness, is to have the surface roughness the same, but instead to have different material for the disc. Examples of materials that could be employed are titanium 64 alloy (105-120 GPa), aluminium (69 GPA), steel (200 GPa), tungsten (400 GPa), silicon carbide (450 GPz). Alternatively, materials such as nylon (4 GPa), PET (2 GPa) or carbon reinforced plastics (50 GPa) could be considered for devices requiring less rigidity/stiffness. This leads to different levels of friction along the longitudinal axis, thus allowing the segment stiffness to be adjusted along the longitudinal axis.

By employing the teachings of the disclosure, the continuum robot can have sections that curve with a circular shape when bent, and other bending shapes, such as quadratic curves. As such, this would provide the robot with better accessibility when navigating within confined spaces, or in situations where extreme control of the arm is required. Continuum robots featuring sections as described above could include being deployed into an aero engine gas path via a borescope port in order to perform precise repair and inspection activities. This could include accurate deployment of a grinding or deposition tool, or a nondestructive testing end effector such as an ultrasonic array or an eddy current probe. Other accurate inspection and or repair activities could be performed in confined environments such as industrial gas turbines, wind turbines or in key hole surgery for example. Furthermore, as these sections are of set design, they can be adjusted on site to improve the performance of the robot arm.

Any of the embodiments discussed above have the benefit that if due to aging or damage the curvature of a section no longer matches that of a circle then stiffening components can be added in appropriate places. Alternatively, the stiffening components can be added to a section formed of three or more segments, in which the joints have the same stiffness to produce a higher stiffness base than the middle, which is again higher than the tip, as in the other embodiments of this disclosure. For example, the stiffening could be done through the addition of struts or through the addition of a sleeve of a certain thickness over a segment to adjust the stiffness of a segment. These segments or sleeves can be made from thermoplastic materials, shape memory alloys and/or shape memory polymers. Alternatively, if the stiffness is too great the robot arm sections do not closely resemble the curvature of a circle, then removal of the stiffening components can be used to reduce the stiffness of the section. Such a design allows for a generic system to be manufactured, but the additional stiffening components allows the robot arm to be used in a number of environments with customised stiffness for each of these sections. Potentially the stiffening components can be made from materials such as shape memory alloys or current/heat activated materials or other materials that will be apparent to the person skilled in the art. The use of sleeves having different stiffness values could be used. This could work for example on sections all having the same stiffness values, such that a sleeve having a highest stiffness is provided around the base segment, a sleeve having less stiffness is provided around the middle segment and a sleeve with the lowest stiffness is provided for the tip segment.

Although the embodiments have been described having three segments the invention is not limited to this. More segments can be added provided that the stiffness decreases from the base of the section to the tip. By using more segments, the control of the curvature may be greater for the trade-off of having a more complex component.

It will be understood that the invention is not limited to the embodiments above-described and various modifications and improvements can be made without departing from the scope of the invention as defined by the appended claims.

## Claims

1. A continuum arm robot section, in which the section comprises at least three segments, featuring a base segment, at least one middle segment and a tip segment, with each segment corresponding to at least one interconnecting portion between pairs of neighbouring discs that form a backbone of the section of the continuum arm robot, wherein the base segment has mg- a greater stiffness than the at least one middle segment and tip segment and the at least one middle segment has a greater stiffness than the tip segment, wherein each segment is made from a compliant and revolute joint pair and **characterised in that** the stiffness of the joints is determined by the surface roughness of the revolute joint.

2. The continuum arm robot section as claimed in claim 1, wherein the stiffness of the joints is determined by the material of the discs within the revolute joints.

3. The continuum arm robot section as claimed in any preceding claim, wherein at least one sleeve is provided around a segment in order to adjust the stiffness of said segment.

4. The continuum arm robot section as claimed in any preceding claim, wherein at least one stiffening member is provided on a segment in order to adjust the stiffness of said segment.

5. A continuum robot having one or more arms, each arm comprising at least one continuum robot arm section as claimed in any preceding claim.

6. Use of a continuum robot as claimed in claim 5 for the inspection and/or repair of a gas turbine engine.

## Patentansprüche

1. Kontinuumsarmroboterabschnitt, bei dem der Abschnitt mindestens drei Segmente umfasst, die ein Basissegment,
mindestens ein Mittelsegment und ein Spitzensegment aufweisen,
wobei jedes Segment mindestens einem Verbindungsteil zwischen Paaren benachbarter Scheiben entspricht, die ein Rückgrat des Abschnitts des Kontinuumsarmroboters bilden, wobei das Basissegment eine größere Steifigkeit aufweist
als das mindestens eine Mittelsegment und Spitzensegment und das mindestens eine Mittelsegment eine größere Steifigkeit als das Spitzensegment aufweist, wobei jedes Segment aus einem nachgiebigen und drehbaren Gelenkpaar besteht und **dadurch gekennzeichnet, dass** die Steifigkeit der Gelenke durch die Oberflächenrauheit des Drehgelenks bestimmt wird.

2. Kontinuumsarmroboterabschnitt nach Anspruch 1, wobei die Steifigkeit der Gelenke durch das Material der Scheiben innerhalb der Drehgelenke bestimmt wird.

3. Kontinuumsarmroboterabschnitt nach einem vorhergehenden Anspruch, wobei um ein Segment herum mindestens eine Hülse vorgesehen ist, um die Steifigkeit des Segments anzupassen.

4. Kontinuumsarmroboterabschnitt nach einem vorhergehenden Anspruch, wobei an einem Segment mindestens ein Versteifungselement vorgesehen ist, um die Steifigkeit des Segments anzupassen.

5. Kontinuumsroboter, der einen oder mehrere Arme aufweist, wobei jeder Arm mindestens einen Kontinuumsroboterarmabschnitt nach einem vorhergehenden Anspruch umfasst.

6. Verwendung eines Kontinuumsroboters nach Anspruch 5 zur Inspektion und/oder Reparatur eines Gasturbinentriebwerks.

## Revendications

1. Section de robot à bras continuum, ladite section comprenant au moins trois segments, possédant un segment de base, au moins un segment médian et un segment d'extrémité, chaque segment correspondant à au moins une partie d'interconnexion entre des paires de disques voisins qui forment la colonne vertébrale de la section du robot à bras continuum, ledit segment de base présentant une plus grande rigidité que ledit au moins un segment médian et ledit segment d'extrémité et ledit au moins un segment médian présentant une plus grande rigidité que le segment d'extrémité, chaque segment étant constitué d'une paire d'articulations souples et tournantes et **caractérisé en ce que** la rigidité des articulations est déterminée par la rugosité de surface de l'articulation tournante.

2. Section de robot à bras continuum selon la revendication 1, ladite rigidité des articulations étant déterminée par le matériau des disques dans les articulations tournantes.

3. Section de robot à bras continuum selon l'une quelconque des revendications précédentes, au moins un manchon étant pourvu autour d'un segment afin d'ajuster la rigidité dudit segment.

4. Section de robot à bras continuum selon l'une quelconque des revendications précédentes, au moins un élément de rigidification étant pourvu sur un segment afin d'ajuster la raideur dudit segment.

5. Robot continuum comportant un ou plusieurs bras, chaque bras comprenant au moins une section de bras de robot continuum selon l'une quelconque des revendications précédentes.

6. Utilisation d'un robot continuum selon la revendication 5 pour l'inspection et/ou la réparation d'un moteur à turbine à gaz.
